# EUROPEAN PATENT APPLICATION

(11) **EP 4 822 704 A1**
(43) Date of publication of application: **14.10.2026**
(21) Application number: 25170175.1
(22) Date of filing: 11.04.2025
(51) Int. Cl.: G16H 10/40, G01N 35/00, G01N 35/04

(54) **COMPUTER-IMPLEMENTED METHOD, COMPUTER PROGRAM, COMPUTER-READABLE MEDIUM, AND DATA PROCESSING SYSTEM**

(71) Applicant: Beckman Coulter, Inc., Brea, CA 92821 (US)
(72) Inventor: REICHLE, Roland, Brea, 92821 (US); BOYAPATI, Karthik, Brea, 92821 (US); MUELLER, Martin, Brea, 92821 (US)
(74) Representative: Maiwald GmbH

(57) **Abstract**

The disclosure relates to a computer-implemented method comprising:
- obtaining a set of requests, wherein each request of the set of requests is a request to carry out an action on a respective sample tube and wherein each request of the set of requests is associated with a respective request priority for that request;
- determining, based on the set of requests, rack attribute data indicative, for each rack of a set of racks contained in a laboratory device, of a respective set of attributes of that rack, wherein each rack of the set of racks is configured to hold one or more sample tubes and holds or is assigned to hold a respective set of sample tubes, and;
- determining, based at least on the rack attribute data, rack priority data indicative, for each rack of the set of racks, of a respective rack priority of that rack; and
- determining, based on the rack priority data, a set of instructions for handling the set of racks contained in the laboratory device.

## Description

The present invention generally relates to the field of handling sample tubes, in particular containing biological fluid samples, e.g., blood samples, in a laboratory automation system. Specifically, the present invention relates to a computer-implemented method, a computer program, a computer-readable medium, and a data processing system.

Various types of clinical tests related to patient diagnosis and therapy can be performed on biological samples by laboratory devices and laboratory automation systems, LAS, (also referred to as: automated laboratory systems ALS). LAS may handle pre-analytical, analytical and/or post-analytical processing of biological samples. Biological samples that can be handled by LASs are in particular body fluid sample such urine, blood, blood plasma, blood serum, interstitial liquid, cerebrospinal liquids, and the like.

In modern laboratory environments, the transfer of sample tubes containing biological specimens between laboratory devices, such as analytical devices and storage devices, presents significant technical challenges. These challenges stem from the need to maintain sample integrity, ensure traceability, and manage complex prioritization requirements in high-throughput workflows.

Some of those challenges are at least partially solved or alleviated by the subject matters of the independent claims of the present disclosure, wherein further examples are incorporated in the dependent claims.

According to an aspect of the present disclosure, there is provided a computer-implemented method, comprising:
- obtaining a set of requests, wherein each request of the set of requests is a request to carry out a respective action on a respective sample tube, wherein each request of the set of requests is associated with a respective request priority for that request;
- determining, based on the set of requests, rack attribute data indicative, for each rack of a set of racks contained in a laboratory device, of a respective set of attributes of that rack, wherein each rack of the set of racks is configured to hold a single sample tube or a plurality of sample tubes and is associated with a respective set of sample tubes, each sample tube of the respective set of sample tubes being held or assigned to be held in said each rack;
- determining, based at least on the rack attribute data, rack priority data indicative, for each rack of the set of racks, of a respective rack priority of that rack; and
- determining, based on the rack priority data, a set of instructions for handling the set of racks contained in the laboratory device.

Each request of the set of requests is a request to carry out the respective action on the respective sample tube, so that each request is associated with the respective action and the respective sample tube. In particular, for each request of the set of requests, the respective sample tube associated therewith is a sample tube held or to be held by a respective rack of the set of racks. According to the present disclosure, an action to be carried out on the sample contained in a sample tube is considered an action to be carried out on the sample tube. Hence, in particular, according to the present disclosure, each request of the set of requests is a request to carry out a respective action on a respective sample tube and/or on a sample contained in the respective sample tube.

For example, each request of the set of requests may be comprised in respective request data, the request data comprising information indicative of that request. For instance, for each request of the set of requests, the respective data may comprise information indicative of the respective action to be carried out and/or of the respective sample tube. In particular, each request of the set of requests may comprise the respective request priority for that request. More particularly, for each request of the set of requests, the respective request data may comprise information specifying the respective request priority.

In particular, the method addresses the prioritization and routing challenges in sample tube handling by determined optimized rack handling instructions based on individual request priorities and rack attributes or, in other words, characteristics.

Obtaining the set of requests may comprise accessing the requests in that set, e.g., retrieving those requests form a computer memory. Retrieving a request may comprise downloading that request. Additionally, or alternatively, obtaining the set of requests may comprise receiving the requests of that set e.g. from the ALS. The two options are not mutually exclusive. For instance, accessing the set of requests may comprise receiving some of the requests and retrieving some other requests.

At least a request, e.g., each request, of the set of requests may be a request to transport a respective sample tube within a laboratory device, from the laboratory device, to the laboratory device, and/or between an automated laboratory system and the laboratory device, i.e., the for that request, the respective action to be carried out is the action of transporting the respective sample tube.

At least a request, e.g., each request, of the set of requests may be a request to transport the respective sample tube to a respective location. The respective location and the respective sample tube may be within the laboratory device. If this is the case, the request requires a transport of the respective sample tube within the laboratory device. For example, in case the laboratory device comprises storage or a line-up for the samples to be analyzed and the respective sample tube is at a location of the storage or line-up, the respective location specified by the request may be another location of the storage or the line-up, e.g., so that the request requires a change in the location within the storage or the line-up e.g. to prioritize the processing of the respective sample tube. Exemplarily, the respective location may be a location of the automated laboratory system and the laboratory device may be connected with but not comprised in, e.g., located outside of, the ALS. That storage device may be connected to the automated laboratory system by a rack handling unit and/or a conveying system for transporting racks with sample tubes between the automated laboratory system and the storage device.

For example, the request priority of each request of the set of priorities indicates a priority category, among a plurality of priority categories, for the processing of the request. For example, the request priority of a request whose respective sample tube contains blood sample of a subject in an emergency room may indicate the priority category indicating the highest priority. For instance, the request priority of a request whose respective sample tube is to undergo a routine cholesterol test may indicate a priority category indicating a priority lower than the highest one. For example, each request of the set of requests includes the respective request priority for that request.

The set of attributes of a rack may comprise one or more of physical and logistical rack attributes. Physical rack attributes can refer to the structural characteristics of racks such as the number of sample tubes it is configured to hold or currently holds, for example. Logistical rack attributes can refer to the operational of functional aspects of the racks within the laboratory workflow, such as the type of actions to be carried out on the sample tube held or assigned to be held by the rack or the number and urgency of requests associated with the sample tubes assigned to be held or held by the racks, for example. An attribute of the set of attributes of the rack may comprise information indicative of one or more of: the rack's content (e.g., the sample tubes held or to be held in the rack), the requests (if any) associated with the sample tubes held or to be held in the rack, the processing requirements for the sample tubes held or to be held in the rack.

Then, the method may use the rack attribute data to determine, e.g., calculate, a rack priority for each rack. The determination may in particular be based on the highest-priority tube in that rack and/or an urgency of tests. The determination may use any algorithm (e.g. weighed algorithm) to balance competing request priorities of sample tubes in the racks. As an example, a rack containing mostly routine samples, but one high-priority sample tubes might be given a higher overall priority to ensure that the high-priority sample is processed swiftly. For example, a high-priority sample tube is a sample tube associated with a request whose rack priority is the highest one, e.g., whose rack priority indicates a priority category indicating the highest priority. In another scenario, racks with earlier expiration time might be prioritized over racks with later expiration time, wherein the expiration time of a rack is the latest among the expiration times of the sample tube held or to be held in that rack.

In particular, determining the set of instructions may comprise generating that set of instructions. For example, the set of instructions is so that the racks of the set of racks are processed according to their rack priorities. In particular, the set of instructions may be so that the racks of the set of racks are processed so that racks with higher priority are processed before racks with lower priority. For example, the set of instructions may be so that the actions to be carried out on the sample tubes held or to be held in racks with higher priority are carried out before the actions to be carried out on the sample tubes held or to be held in racks with lower priority. Exemplarily, the set of instructions is so that at least the racks of a subset of the set of racks are processed according to their rack priorities. In particular, the set of instructions may be so that the racks of that subset of racks are processed so that racks with higher priority are processed before racks with lower priority. For example, the set of instructions may be so that the actions to be carried out on the sample tubes held or to be held in racks of that subset with higher priority are carried out before the actions to be carried out on the sample tubes held or to be held in racks of that subset with lower priority.

In some examples, determining the set of instructions may comprise determining a handling priority of the racks of the set of racks, wherein determining the handling priority is based on the rack priority data. In some cases, determining the handling priority may be based on further factors or constraints, such as requirements on the turnaround time of (some of) the sample tubes held or to be held in the racks of the set of racks or on the throughput of the automated laboratory system or of the laboratory. In those cases, determining the handling priority may be carried out by using a Pareto optimization aiming at simultaneously optimizing a plurality of objective functions. In particular, the plurality of objective functions comprises a first objective function and further objective functions. The first objective function implements the requirement that the handling priority is to comply with the rack priorities and further objective functions and each objective function of the further objective functions implement further constraints.

The set of instructions may include rack handling sequences minimizing priority conflicts. The rack handling sequences may for example include rack routing sequences, i.e. sequences on how to handle one or more racks within the laboratory device and/or between the automated laboratory system and the laboratory device. However, the method may not only generate instructions to route a high-priority rack to an available analyzer but simultaneously comprise ordering the reconfiguration of a lower-priority rack to optimize its sample distribution, e.g., by moving sample tubes of a certain priority to another rack. In another scenario, the instructions may instruct the rack handling unit to transfer specific sample tubes from one rack to another rack to create a rack with high-priority sample tubes for immediate processing.

The computer-implemented method may be partially or fully computer-implemented, e.g., one, several or all of the steps of the method may be executed by a computing system. The computing system may comprise one or more computing devices, which may be spatially distributed. A computing device may comprise one or more processors and one or more memories, which e.g., may store computer-executable instructions for the execution of the method. Hence, different steps of the method may be carried out by the same or different computing devices.

Additionally, the method may comprise steps executed by different hardware than a computing system, e.g., a conveyor system for transferring racks between different laboratory devices, a rack handling unit for handling the racks, or a rack building unit for providing sample tubes to the racks and/or removing them from the racks. Those units may be handling the distribution of sample tubes among different racks. In particular, the rack building unit may build racks by providing sample tubes to the racks and/or removing them from the racks. For this purpose, the rack handling or building unit may comprise a robotic arm. The rack handling or building unit may execute the set of instructions.

The laboratory automation system may be configured for at least partial or fully automated handling of sample tubes for laboratory purposes such as sample analysis for clinical testing. In particular, according to the present disclosure, an automated laboratory system may be an assembly comprising a plurality of components. The ALS may comprise a computing device operatively connected to these components and is configured to control each component. A component of the ALS may be an analytic instrument, a pre-analytic instrument, a post-analytic instrument, or a transportation component (e.g., a track, a belt, a tube carrier and the like) configured to move a sample tubes. An analytic instrument (hereinafter referred to also as: analyzer) may be configured to carry out at least a clinical test. A pre-analytic instrument may be configured to carry out one or more pre-analytic steps on a biological sample to prepare the biological sample for the analytic instrument(s). A pre-analytic instrument may include or consist of a centrifuge, a decapper, an input module and/or an aliquoter. A post-analytic instrument may be configured to carry out one or more post-processing steps on the biological sample after the biological sample has been processed by one or more laboratory analyzers. A post-analytic instrument may include or consists of a recapper, a storage unit, in particular a refrigerated storage unit, and an output module.

According to the present disclosure, a sample may be a biological sample, in particular a sample is a sample of a bodily fluid of a human or animal subject. For example, the bodily fluid may be a physiological fluid, such as blood, saliva, urine, sweat, amniotic fluid, cerebrospinal fluid, ascites fluid, or the like. The sample may comprise one or more components that may potentially comprise analytes of interest for performing at least one clinical test. For instance, blood serum, blood cells and blood plasma are components of a blood sample.

According to the present disclosure, a clinical test may comprise one or more procedures that, when carried out on the biological sample or on a component thereof, allow for estimating the value of a parameter, e.g. a clinical parameter. In particular, a clinical test may comprise physical, biological, optical, mechanical, immunological, and/or chemical procedures.

The sample tube may be configured to receive and hold a biological sample. Accordingly, the sample tube may comprise an opening for receiving the sample and a bottom as well as one or more walls to hold the sample, wherein the opening may be configured to be releasably closed by a closing element, e.g., a cap. In particular, the sample tube may have a substantially cylindrical shape (i.e., a cylindrical wall), with the opening at one base and the bottom at the opposite base.

The racks are holding units in any physical shape or form capable of holding several sample tubes, e.g. in the range of 2 to 100, in particular in the range of 4 to 60 or in the range of 6 to 40. The racks may comprise receptacles for receiving and holding each sample tube individually. According to the present invention, a rack may be a single-tube holder, i.e., a tube holder configured to hold a single sample tube at a time.

The set of instructions may comprise one or more of: at least an instruction for transferring one or more racks of the set of racks between the automated laboratory system and the laboratory device; at least an instruction for transferring one or more racks of the set of racks within the laboratory device; at least an instruction for transferring one or more racks of the set of racks between the laboratory device and another laboratory device, e.g., a standalone analyzer not comprised in the laboratory automation system; at least an instruction to keep one or more racks of the set of racks in an idle state, e.g., in which they are not processed; at least an instruction for removing or disposing sample tubes from one or more racks of the set of racks; at least an instruction for moving one or more sample tubes from one rack of the set of racks to another rack of the set of racks; at least an instruction for moving a sample tube from a single-tube holder to a rack of the set of racks; at least an instruction for disposing one or more sample tubes held in a rack of the set of racks; and at least an instruction for moving a sample tube from a rack of the set of racks to a single-tube holder. Alternatively, or in conjunction, the set of instructions may comprise at least an instruction for carrying out a clinical test on the sample contained in a sample tube held or to be held in a rack of the set of racks.

In particular, if the laboratory device is a storage device, the set of instructions may comprise at least an instruction for transferring one or more racks of the set of racks between the storage device and the automated laboratory system, where they can be tested on one or more analyzers. In another example, the set of instructions may comprise at least an instruction for transferring one or more racks of the set of racks within the storage device, such that the location of the rack within the storage device is changed in accordance with the rack priority.

For example, the set of instructions may comprise an instruction for moving a sample tube, e.g., a high-priority sample, from a rack to a single-tube holder or vice versa (e.g., after a high-priority test has been performed on the sample included in that of a sample tube).

Specifically, the set of instructions may comprise at least an instruction for transferring one or more racks of the set of racks within the laboratory device. Such transfer can also be referred to as an inter-device transfer within the laboratory device.

The typical use of standardized racks for storing and transporting multiple sample tubes allows high-throughput workflows. The inventors, however, have found that this use may lead to conflicts in the priority of the different sample tubes on a rack and, in particular, that the loading to and/or retrieval from a storage device of racks containing mixed-priority samples may be suboptimal. In particular, due to rack-based processing racks must typically be moved as a unit, making it difficult to prioritize individual high-priority samples: urgent samples may be delayed if they are part of a rack containing mostly routine samples, as the system may not be able to easily or quickly extract and expedite the handling of single sample tubes. This may lead to delays and/or inefficiencies in the loading to and/or retrieval from a storage device of sample tubes. The present disclosure provides a solution which considers the individual sample tube prioritization while maintaining the throughput benefits of rack-based transport. Specifically, the method according to the present disclosure allows for handling racks according to their priority, which is obtained by considering the priority of the requests for the sample tubes held or to be held in those racks. This way, granular priority information on the requests for the sample tubes is considered and the rack-based processing, according to which an entire rack is handled as a whole, is performed by taking into account the priority of the requests for the sample tubes thereby reducing conflicts in the priority of the different sample tubes held in the racks. Hence, the efficiency of the handling of the racks may be improved and the priority of the requests may be addressed without the need for handling sample tubes singularly and/or rearranging the sample tubes in the racks.

The laboratory device may comprise a first unit and a second unit, wherein the first unit of the laboratory device is configured to contain one or more racks of the set of racks so that sample tubes contained in at least one of those one or more racks are transferable between the automated laboratory system and the laboratory device, e.g., the second unit, and wherein the second unit of the laboratory device is configured to contain one or more racks of the set of racks and to process and/or store sample tubes contained in those one or more racks. For example, the first unit may be a rack building or handling unit, which may process or handle the sample tubes, and the second unit may be a storage unit. Typically, the first unit would have less space for containing racks compared to the second unit, such that an efficient handling management of racks is required according to the determined rack priorities. Accordingly, an inter-device transfer as described above is advantageous.

In some examples, determining the set of instructions may be based on a set of handling criteria. In particular, the set of instructions may be determined so that at least a set of handling criteria or, in other words, handling requirements are fulfilled. More particularly, the set of instructions may be determined so that each handling requirement of the set of handling requirement are fulfilled. For example, a handling requirement may be the requirement that, after the set of racks are handled according to the set of instructions, the racks with highest priority are positioned in the first unit. For instance, a handling requirement may be the requirement that, after the set of racks are handled according to the set of instructions, the lowest among the rack priorities associated with the racks in the first unit is higher than or equal to the highest among the rack priorities associated with the racks in the second unit. In particular, a handling requirement may be the requirement that, after the set of racks are handled according to the set of instructions, at least one of the following conditions are fulfilled:
∘ condition 1: the rack priority of each rack in the first unit is higher than a given threshold; and
∘ condition 2: the rack priority of each rack in the second unit is lower than or equal to the given threshold.

Exemplarily, the set of instructions may comprise information specifying a first set of racks and a second set of racks, wherein the first set of racks consists of the racks of the first unit that are to be moved to the second unit and the second set of racks consists of the racks of the second unit that are to be moved to the first unit. In particular, determining the set of instructions may comprise determining, among the set of racks the N racks with highest rack priority, wherein N is a positive integer lower than or equal to the number of racks that the first unit may accommodate. determining the set of instructions may further comprise determining, among the N racks with highest rack priority, the second set of racks which consists of the Q racks comprised in the second unit, wherein Q is a positive integer lower than or equal to N. The first set of racks consists of the Q racks comprised in the first unit which are not among the N racks with highest rack priority.

The set of instructions may comprise one or more of: at least an instruction for transferring one or more racks of the set of racks from the first unit to the second unit; and at least an instruction for transferring one or more racks of the set of racks from the second unit to the first unit. Accordingly, for example, the set of instructions may be handling which of one or more racks of the set of racks is to be held in the limited space of the rack building unit or generally to be handled thereby and which of the other racks of the set of racks are to be kept in the storage unit based on the rack priority data.

Exemplarily, each request of the set of request is associated with respective action data. The respective action data associated with that request may comprise information indicative of the respective action associated with said each request. For example, for at least a request of the set of requests, the respective action may be: (i) the action of loading the respective sample tube to the laboratory device, e.g., to the storage unit, (ii) the action of retrieving the respective sample tube from the laboratory device, e.g., from the storage unit, (iii) the action of manually removing the respective sample tube from the laboratory device, e.g., from the storage unit, or (iv) the action of disposing, e.g., automatically disposing, the respective sample tube. In particular, for at least a request of the set of request, the respective action associated therewith may be the action of carrying out a clinical test on the sample contained in the respective sample container.

For each rack of the set of racks, the respective set of attributes may comprise one or more of: a first attribute indicative of whether at least one request of the set of requests is a request to carry out an action on a sample tube held or assigned to be held by said each rack; a second attribute indicative of a cardinality of a respective subset of the set of requests, each request of the respective subset being a request to carry out a respective action on a respective sample tube held or assigned to be held by said each rack; a third attribute indicative of a respective time at which a respective first request was obtained, wherein the respective first request is, among the requests of the respective subset, a request with highest priority; and a fourth attribute indicative of the respective action associated with the respective first request. Herein, for each rack of the set or racks, the requests of the respective subset of the set of requests may also be referred to as pending requests of said each rack. According to the present disclosure, for each rack of the set or racks, the respective first request may also be referred to the highest priority pending request of that rack.

The first attribute can address the issue of priority conflicts in rack processing by identifying racks requiring immediate action. This attribute, when used for the determination of the rack priority data, can help to determine whether a rack contains at least one sample tube with a pending request, allowing for more efficient prioritization. For example, a rack holding ten sample tubes might have this attribute set to "true" if even one tube has a pending request. Consequently, this rack could be prioritized over others with no pending requests and hence have a higher rack priority assigned. As consequence, the set of instructions may ensure that racks with pending requests are handled with higher priority than the racks without pending transfer requests.

The second attribute may specify the number of pending requests for sample tubes in a rack, helping to address throughput inefficiencies by quantifying the number of pending requests per rack. For instance, a rack with eight tubes might have five pending requests, including three high-priority tests and two routine tests. In this case, the second attribute could be set to "five" signaling higher priority compared to a rack with only two pending requests, whose second attribute is set to "two". Considering the second attribute when determining the rack priority thus helps to provide instructions that consider the number of the pending requests per rack.

The third attribute may be a time stamp and allow for handling time-sensitive samples and ensuring that high-priority pending requests are not delayed. For example, if a rack holds ten tubes, and the highest-priority pending request for this rack (such as a critical cardiac marker test) was logged on February 12, 2025 at 8 am, the third attribute for this rack may be equal to 2025.02.12.08:00. This rack may have higher priority compared to a rack whose highest-priority pending request was logged on February 12, 2025 at 1 pm and hence whose third attribute is equal to 2025.02.12.13:00.

The fourth attribute allows for determining the rack priority by considering the actions associated with the pending requests. For example, higher rack priorities could be assigned to actions that are generally or in a current situation particularly relevant. For example, if storage space is low in a storage device, a higher rack priority can be assigned to racks with at least a pending request associated with one of the actions (ii) to (iv) described above.

Any two or more of the above attributes can be also used synergistically to optimize rack handling within the laboratory workflow. For example, the rack priority of a rack with second attribute equal to five and third attribute 2025.02.12.08:00 is higher than the priority of a rack with second attribute equal to five and third attribute 2025.02.12.13:00 and the latter priority is in turn higher than the priority of a rack with second attribute smaller than five, irrespective of the value of the third attribute of the latter rack.

Determining the rack attribute data may comprise one or more of:
∘ accessing sample tube information indicative of, for each rack of the set of racks, the sample tubes that are held or to be held in said each rack;
∘ determining, for each rack of the set of racks, the number of the pending requests for said each rack;
∘ determining, for each rack of the set of racks with at least a pending request, the respective first request;
∘ determining, for each rack of the set of racks with at least a pending request, the respective time at which the respective first request was obtained;
∘ determining, for each rack of the set of racks with at least a pending request, the respective action associated with the respective first request;
∘ for each rack of the set of racks, using one or more of: the sample tube information and the number of the pending requests for said each rack, to generate the respective first attribute and/or the respective second attribute; and
∘ for each rack of the set of racks with at least a pending request, using one or more of: the respective first request, the respective time at which the respective first request was obtained, and the respective action associated with the respective first request to generate the respective third attribute and/or the respective fourth attribute.

Determining the rack priority data may comprise, for each rack of the set of racks, increasing or decreasing the rack priority depending on: a number of sample tubes which are held or assigned to be held by that rack and which are subject to at least one request in the set of requests; a number of requests in the set of requests which are requests to carry out actions on one or more sample tubes held or assigned to be held by that rack; a cardinality of one or more requests in the set of requests which the sample tubes held or assigned to be held by that rack are subject to; a time of obtaining one or more requests in the set of requests which the sample tubes held or assigned to be held by that rack are subject to; and an action associated with one or more requests in the set of requests which the sample tubes held or assigned to be held by that rack are subject to. In particular, the rack priority data may be increased or decreased depending on any one or more of the above-described attributes.

Exemplarily, determining the rack priority data comprises determining, for at least a rack of the set of racks, e.g., for each rack of the set of racks, the respective rack priority such that the respective rack priority when at least one request of the set of requests is a request to carry out an action on a sample tube held or assigned to be held by said rack is higher than the respective rack priority when no request of the set of requests is a request to carry out an action on a sample tube held or assigned to be held by said rack. For example, the rack priority of a rack with at least a pending request would be higher than the rack priority that the same rack would have if it had no pending requests.

In particular, determining the rack priority data comprises determining, for each rack of the set of racks, the respective rack priority such that the rack priority of each rack with at least a pending request (i.e., each rack such that at least one request of the set of requests is a request to carry out an action on a sample tube held or assigned to be held by said each rack) is higher than the rack priority of each rack with no pending requests (i.e., each rack such that no requests of the set of requests is a request to carry out an action on a sample tube held or assigned to be held by said each rack).

In some examples, determining the rack priority data comprises determining, for at least a rack of the set of racks, e.g., for each rack of the set of racks, the respective rack priority such that:
∘ the respective rack priority increases as the cardinality of a respective subset of the set of requests increases; and/or
∘ the respective rack priority increases as the respective time at which the respective first request was obtained decreases.

In particular, determining the rack priority data comprises determining, for at least a rack of the set of racks, e.g., for each rack of the set of racks, the respective rack priority such that:
∘ the respective rack priority when the respective action associated to the first request is the action of loading the respective sample tube to the laboratory device is higher than when the respective action associated to the first request is an action different from the action of loading the respective sample tube to the laboratory device; or
∘ the respective rack priority when the respective action associated to the first request is the action of retrieving the respective sample tube from the laboratory device is higher than when the respective action associated to the first request is an action different from the action of retrieving the respective sample tube from the laboratory device.

In some examples, determining the rack priority data may comprise determining, for at least a rack of the set of tracks, e.g., for each rack of the set of racks, the respective rack priority by using a lookup table and/or a decision tree. In particular, the lookup table and/or the decision tree may be configured to output a rack priority for a rack by using one or more of: the first attribute, the second attribute, the third attribute, and the fourth attribute of that rack. For example, the lookup table and/or the decision tree may depend on the position of the rack within the laboratory device, e.g., whether the rack is in the first unit or in the second unit.

The laboratory device may be one or more of: a laboratory analyzer, a pre-analytic laboratory instrument, and a post-analytic laboratory instrument. Similarly, the automated laboratory system may comprise any one or more of these types of laboratory devices.

The laboratory device may be a storage device for storing sample tubes. Such storage device may have added capability such as for freezing the sample tubes contained therein. The storage device may have limited space inside the storage device for storing the racks with the sample tubes therein. The storage device may comprise different storage spaces among which the racks may be distributed in accordance with their rack priorities.

Determining the rack attribute data may comprise determining, for each rack of the set of racks, the respective set of attributes of that rack, based on the request priority of each request of the set of requests which is a request to carry out an action on a sample tube of the respective set of tubes held or assigned to be held by that rack. Additionally, or alternatively, determining the rack priority data comprises determining, for each rack of the set of racks contained in the laboratory device, the respective rack priority of that rack based on the respective set of attributes of that rack.

The method may further comprise causing the set of racks to be handled according to the set of instructions. Causing the laboratory device to handle the racks of the set of racks according to the set of instructions may comprise instructing the laboratory device to handle the racks of the set of racks according to the set of instructions. In particular, causing the laboratory device to handle the racks of the set of racks according to the set of instructions may comprise handling, e.g., by the ALS or by the laboratory device, the set of racks according to the set of instructions. In this case, the laboratory device may for example comprise the already mentioned rack building or handling unit for transferring racks or single sample tubes among racks.

According to a further aspect, there is provided a computer program comprising instructions which, when executed by a computer, cause the computer to carry out the method of this disclosure.

According to a further aspect, there is provided a computer-readable medium comprising instructions which, when executed by a computer, cause the computer to carry out the method of this disclosure.

According to a further aspect, there is provided a data processing system, i.e., a computing device, comprising a processor configured to carry out the method of the present disclosure. For example, the data processing system may comprise one or more computing devices, in particular of or connected to the laboratory automation system. The data processing system may also comprise the computer program and/or the computer-readable medium of this disclosure.

According to the present disclosure, a computing device may comprise one or more processors and one or more memories, which may store computer-executable instructions which when executed by a computer, cause the computer to carry out the method of this disclosure.

According to yet another aspect of this disclosure, there may be provided a laboratory automation system and/or a laboratory device comprising the computer program, the computer-readable medium and/or the data processing system of this disclosure.

It is noted that the above aspects, examples and features may be combined with each other irrespective of the aspect involved.

The above and other aspects of the present disclosure will become apparent from and elucidated with reference to the examples described hereinafter.

Exemplary embodiments will be further described with reference to Figures, wherein:
Figure 1 shows a schematic illustration of a laboratory automation system and a laboratory device.
Figure 2 shows a schematic illustration of a data processing system.
Figure 3 shows a schematic illustration of a rack with sample tubes.
Figure 4 shows a schematic illustration of a method for handling racks.
Figure 5a shows a schematic illustration of a first decision tree for determining the rack priority of a rack.
Figure 5b shows a schematic illustration of a second decision tree for determining the rack priority of a rack.
Figure 6a shows a schematic illustration of a first arrangement of racks within the laboratory device.
Figure 6b shows a schematic illustration of a second arrangement of racks within the laboratory device.
Figure 7 shows a schematic illustration of a third decision tree for determining the rack priority of a rack.

The Figures are schematic only and not true to scale. In principle, identical or like parts, elements and/or steps are provided with identical or like reference numerals in the Figures.

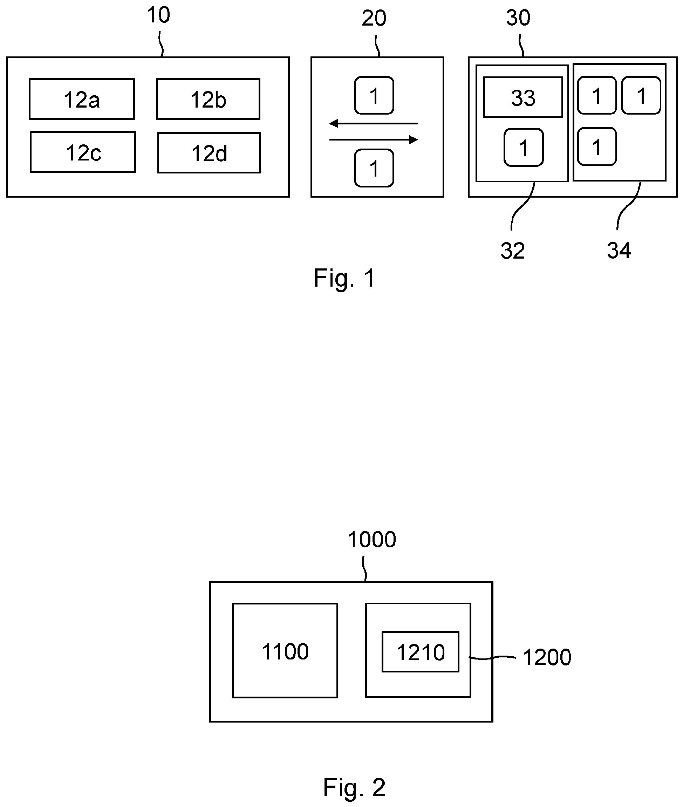

**Fig. 1** shows a schematic representation of a laboratory automation system (LAS) 10 and a laboratory device 30. The laboratory automation system 10 may be configured for at least partial or fully automated handling of sample tubes for laboratory purposes such as sample analysis for clinical testing. The automated laboratory system may comprise a plurality of components 12a-12b. The LAS 10 may comprise one or more a pre-analytic instruments 12a, one or more analytic instruments (i.e., analyzers) 12b, 12c, and or more post-analytic instruments 12d. The one or more pre-analytic instruments 12a are configured to carry out one or more pre-analytic steps on a biological sample and/or on the tube comprising that biological to prepare the biological sample for testing. The one or more pre-analytic instruments 12a may comprise a centrifuge, a decapper, an input module and/or an aliquoter. The one or more analyzers 12a, 12b may be configured to carry out at least a clinical test. The one or more analyzers 12a, 12b may comprise a hematology analyzer, a urinalysis analyzer, a clinical chemistry analyzer, an immunoassay analyzer, and the like. The one or more post-analytic instruments 12d are configured to carry out one or more post-processing steps on a biological sample and/or the tube comprising that biological sample. The one or more post-analytic instruments 12d may comprise a recapper, a storage unit, in particular a refrigerated storage unit, and/or an output module.

The LAS 10 may comprise a transportation component (not shown) configured for moving sample tubes from one instrument 12a-12d of the LAS 10 to another instrument 12a-12d of the LAS 10. The automation components may include one or more tracks, one or more belts and/or container carriers configured to move biological samples contained in sample containers. The LAS 10 may comprise a computing device (not shown) which may be in data communication one or more of the instruments 12a-12d of the LAS 10 and configured to control those instruments 12a-12d. for instance, the computing device may be or comprise the data processing system schematically depicted in fig. 2.

The laboratory device 30 may comprise a first unit 32 and a second unit 34. The first unit 32 is configured to contain one or more racks 1 so that sample tubes contained in at least one of those racks 1 are transferable between the automated laboratory system 10 and the laboratory device 30, e.g., the second unit 34 thereof. The first unit 32 is configured transfer racks 1 between the automated laboratory system 10 and the laboratory device 30, as well as within the laboratory device 30 itself, e.g., by using the robotic arm 32. For instance, the robotic arm 33 is configured to pick, place, and retrieve sample tubes or racks 1. In particular, the racks 1 are transferred between the automated laboratory system 10 and the laboratory device 30 via a transfer unit configured to facilitate the transfer of racks between the automated laboratory system 10 and the laboratory device 30. The second unit 34 of the laboratory device 30 is configured to contain one or more racks of the set of racks and to process and/or sample tubes contained in those one or more racks.

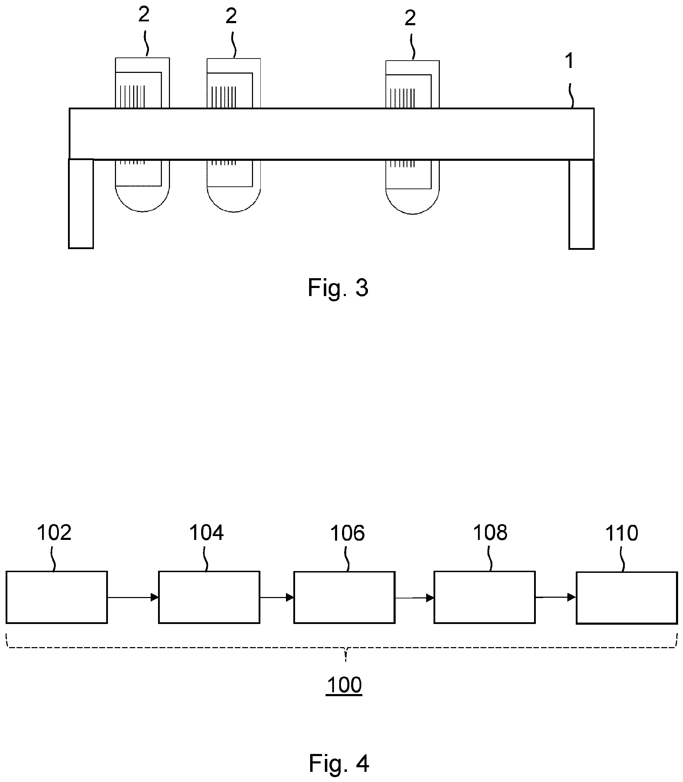

For example, laboratory device 30 may be a storage device 30. If this is the case, the first unit 32 may be a rack building or handling unit, which may process or handle the sample tubes. The second unit 34 may be a storage unit. The racks 1 that the laboratory device 30 is configured to comprise one or more tubes. An exemplary rack which the laboratory device 30 is configured to contain is schematically depicted in fig. 3. The first unit, (e.g., the rack handling unit) 32 may comprise some limited storage space for storing racks 1, e.g., one or two racks 1, whereas the first unit (e.g., the storage unit) 34 may be configured for longer storage and accordingly may be configured with more storage space, e.g., to store three or more, in particular significantly more such as tens or hundreds of racks 1. Merely exemplary, only three racks are shown in the second unit 34 in Fig. 1.

The laboratory device 30 may comprise a computing device (not shown) configured to instruct and/or control the robotic arm 33 to move racks between the first unit 32 and the second unit 34 and to move racks between the first unit 32 and the transfer unit 20. Exemplarily, the computing device comprised in the laboratory device 30 may be or comprise the data processing system schematically depicted in fig. 2. In particular, the computing device comprised in the laboratory device 30 is in data communication with the computing device comprised LAS 10.

In particular, the LAS 10 operates by receiving requests for sample tube transfers, prioritizing them based on urgency, and executing instructions to handle the racks 1 accordingly. For example, if an urgent test is required for a certain sample tube, the system can identify the relevant rack 1 in the rack handling unit 32 or storage unit 34, retrieve it using the robotic arm 33, and transfer it to one of the laboratory devices 12 of the LAS 10 via the transfer unit 20.

**Fig. 2** shows a schematic illustration of a data processing system 1000 which, e.g., is used to carry out the computer-implemented method 100 shown in Fig. 4. The data processing system 1000 includes a processor 1100 and a computer-readable medium, e.g., a computer memory, 1200. The data processing system may comprise an input/output (I/O) interface (not shown), and a network interface controller (not shown). One or more peripheral (input or output) devices (not shown) may be connectable to the input/output interface. The peripheral input devices may include one or more of the following: a computer keyboard and/or a pointing device, such as a mouse. The peripheral output devices may include one or more of the following: a terminal, a printer, a disk drive or other storage device, and a video monitor. The processor 1100 may include a central processing unit (CPU) and/or a graphics processing unit (GPU), each having one or more processing cores. The computer-readable medium 1200 may include primary memory, such as a random access memory (RAM), read-only memory (ROM) or flash memory, and/or secondary memory, such as a solid-state drive or a hard disk drive. The network interface controller may be connectable to a computer network. In particular, if the data processing system 1000 is comprised in the laboratory device 30 schematically depicted in fig. 1, the computing device may be in data communication with the computing device comprised in the LAS 10 schematically depicted in Fig. 1, e.g., via its network interface controller. Alternatively, if the data processing system 1000 is comprised in the LAS 10 schematically depicted in Fig. 1, the computing device may be in data communication with the computing device comprised in the laboratory device 30 schematically depicted in fig. 1, e.g., via its network interface controller.

The processor 1100 of the data processing system 1000 is configured to carry out the method of the present disclosure. The computer-readable medium 1200 comprises instructions which, when executed by the data processing system 1000, cause the data processing system 1000 to carry out the method of this disclosure. In particular, the computer-readable medium 1200 stores the computer program according to the present disclosure.

**Fig. 3** shows an exemplary design of a rack 1 configured to hold a plurality of sample tubes 2. The rack 1 may be configured with receptacles, each receptacle being configured to securely hold a single sample tube 2. The sample tubes 2 may contain biological fluids such as blood, urine, or cerebrospinal fluid. The receptacles of the rack 1 may be arranged along a single column and may be equally spaced with respect to one another. Alternatively, the receptacles of the rack 1 may be arranged in grid comprising R rows and C columns, wherein R and C are positive integers greater than one. According to the present disclosure, a rack may be a single-tube holder, i.e., a rack configured to hold a single tube at a time.

**Fig. 4** illustrates a flowchart of an exemplary method 100 according to the present disclosure. In particular, the method 100 may be carried out by the data processing system 1000 schematically depicted in fig. 3. The method 100 comprises obtaining the set of requests 102. Each request of the set of requests is a request to carry out a respective action on a respective sample tube (e.g., for some requests, on a respective sample contained in that respective sample tube). For example, at least a request, e.g., each request, of the set of requests is a request to transport the respective sample tube within the laboratory device 30, specifically between the rack handling unit 32 and the storage unit 34, and/or between the automated laboratory system 10 and the laboratory device 30. Each request of the set of requests includes a respective request priority, which indicate the urgency and/or importance of that request. For example, if the laboratory device is a storage device, the following hold:
∘ The priority associated with a request to load a sample tube in the storage device ("load to storage" request) is higher than the priority associated with a request to retrieve a sample tube from the storage device ("retrieve from storage" request);
∘ The priority associated with a retrieve from storage request is higher than the priority associated with a request to manually remove a sample tube from the storage device ("manual removal" request); and
∘ the priority associated with a manual removal request is equal to the priority associated with a request to automatic dispose a sample tube contained in the storage device ("automatic disposal" request).

The method further comprises determining, based on the set of requests, rack attribute data 104. The rack attributed data are indicative, for each rack of the set of racks, of a respective set of attributes of that rack. For example, determining the rack attribute data may comprise determining, for each rack of the set of racks, the following attributes:
∘ Attribute A1, indicative of the location of that rack, e.g., whether that rack is located in the storage unit or in the rack handling unit;
∘ Attribute A2, indicative of the number of pending requests of that rack (i.e., the respective cardinality of a respective subset of the set of requests, each request of the respective subset being a request to carry out an action on a respective sample tube held or assigned to be held by that rack);
and, if the number of pending requests of that rack is not zero:
o Attribute A3, indicative of the action associated with the highest priority pending request of that rack (highest priority pending request of that rack being the first request, i.e., the transfer request with highest priority among the requests of the respective subset).

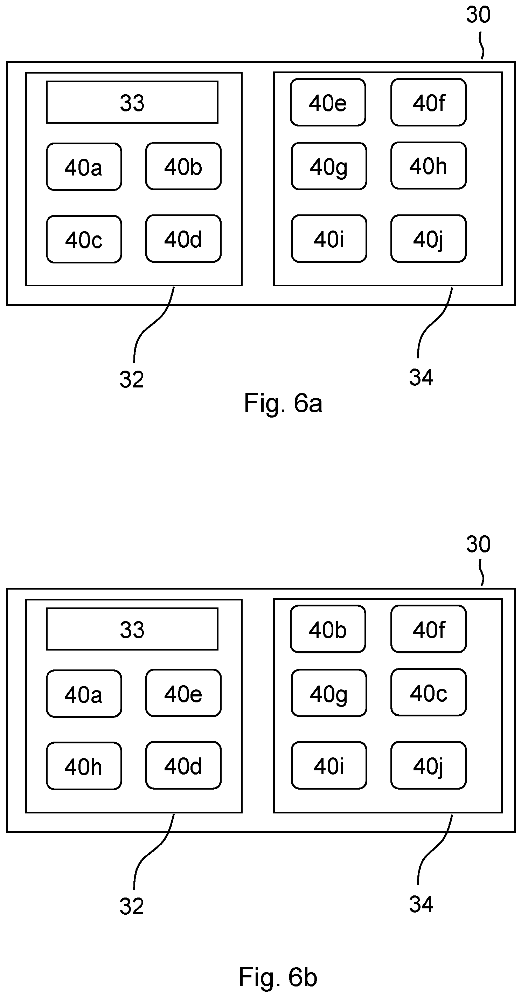

**Fig. 6a** schematically depicts a laboratory device 30 according to the present disclosure. In some examples, the laboratory device 30 comprises 10 racks 40a-40j.

In some examples, the laboratory device 30 is a storage device. In those examples, in the rack configuration depicted in Fig. 6a, four racks 40a-40d are contained in the rack handling unit 32 and six racks 40e-40j are contained in the storage unit 34. With reference to Fig. 6a, for instance, a first sample tube associated with a request to load the first sample tube in the storage device 30 is to be held in the rack 40a. Moreover, the rack 40a holds (i) a second sample tube associated with a request to retrieve that sample tube from the storage device and (ii) a third tube that is not associated to any pending request. If this is the case, the attributes of the rack 40a read as follows:
∘ A1 = "Rack handling unit"
∘ A2 = "2"
∘ A3 = "Load to storage"

For example, the attributes of the racks 40a-40j, may be summarized in the following table:

**Table 1a**

| Rack | Attribute A1 | Attribute A2 | Attribute A3 |
|---|---|---|---|
| 40a | Rack handling unit | 2 | Load to storage |
| 40b | Rack handling unit | 0 | - |
| 40c | Rack handling unit | 1 | Manual Removal |
| 40d | Rack handling unit | 1 | Retrieve from storage |
| 40e | Storage unit | 3 | Load to storage |
| 40f | Storage unit | 0 | - |
| 40g | Storage unit | 0 | - |
| 40h | Storage unit | 2 | Retrieve from storage |
| 40i | Storage unit | 4 | Automatic disposal |
| 40j | Storage unit | 0 | - |

In some other examples, the laboratory device 30 is an analyzer. In those examples, in the rack configuration depicted in Fig. 6a, four racks 40a-40d are contained in the rack handling unit 32 and six racks 40e-40j are contained in the analyzing unit 34. With reference to Fig. 6a, for instance, the rack 40a holds (i) a sample tube associated with a request to retrieve that sample tube from the laboratory device and (ii) a sample tube that is not associated to any pending request. If this is the case, the attributes of the rack 40a read as follows:
∘ A1 = "Rack handling unit"
∘ A2 = "1"
∘ A3 = "Retrieve from the laboratory device"

In those other examples, for instance, the attributes of the racks 40a-40j, may be summarized in the following table:

**Table 1b**

| Rack | Attribute A1 | Attribute A2 | Attribute A3 |
|---|---|---|---|
| 40a | Rack handling unit | 1 | Retrieve from the laboratory device |
| 40b | Rack handling unit | 2 | Carry out an urgent test |
| 40c | Rack handling unit | 2 | Carry out a non-urgent test |
| 40d | Rack handling unit | 0 | - |
| 40e | Analyzing unit | 2 | Retrieve from the laboratory device |
| 40f | Analyzing unit | 3 | Carry out a non-urgent test |
| 40g | Analyzing unit | 1 | Carry out a non-urgent test |
| 40h | Analyzing unit | 0 | - |
| 40i | Analyzing unit | 4 | Carry out an urgent test |
| 40j | Analyzing unit | 2 | Carry out an urgent test |

The method further comprises determining, based at least on the rack attribute data, rack priority data 106. The rack priority data indicates, for each rack, a respective priority level that dictates its handling order within the laboratory device. Determining the rack priority data may comprise determining, for each rack of the set of racks, the respective rack priority by using a lookup table and/or a decision tree. In particular, the lookup table and/or the decision tree may be configured to output the rack priority of a rack by using the attributes of that rack.

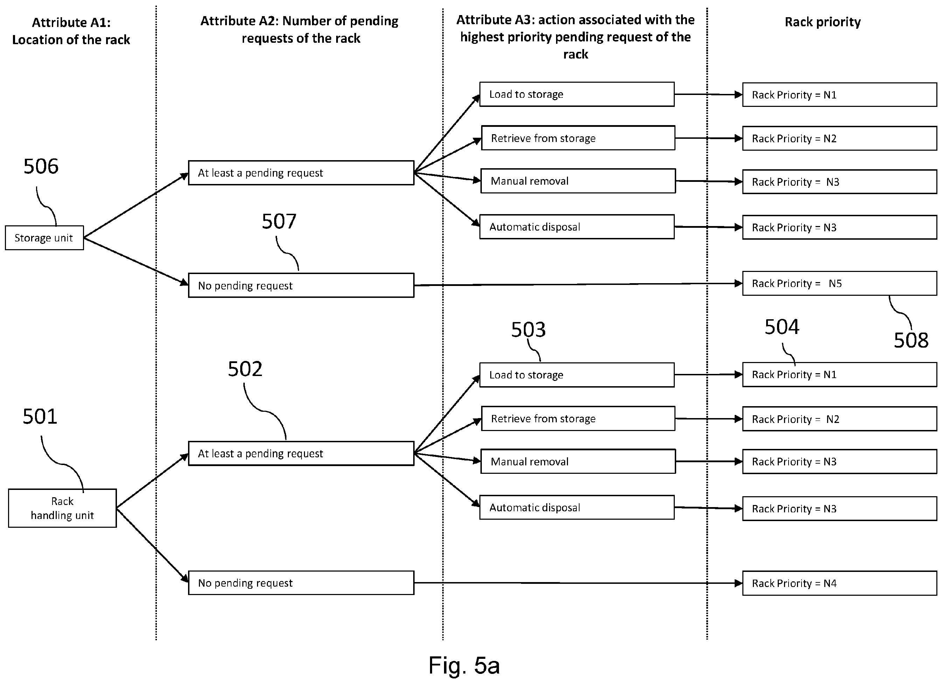

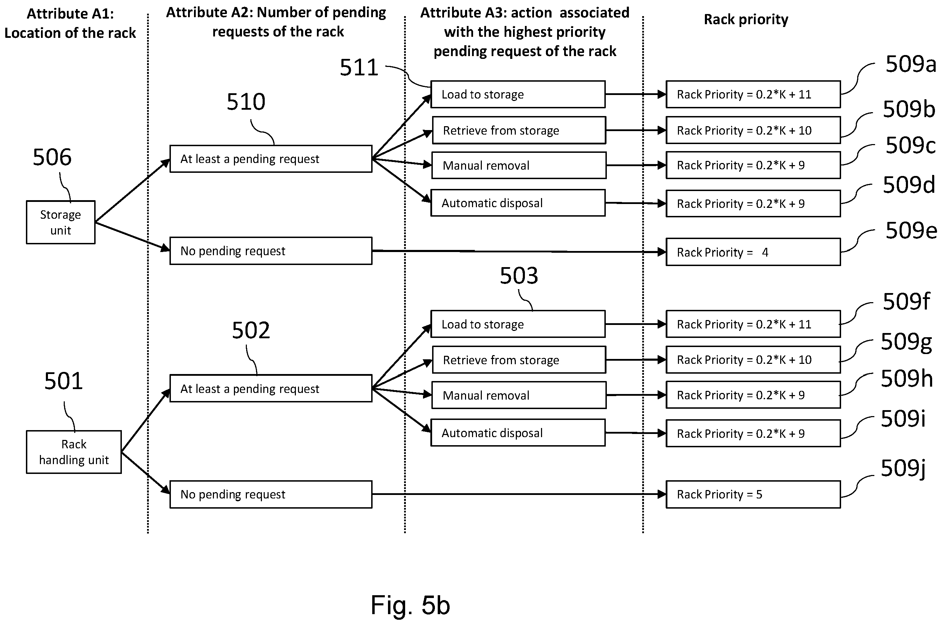

**Fig. 5a** and **Fig. 5b** schematically depict a first and a second exemplary decision tree according to the present disclosure, respectively. In particular, those decision trees are configured to output the rack priority of a rack by using the attributes of that rack e.g., when the laboratory device 30 is a storage device.

Fig. 5a depicts a first decision tree for calculating rack priority based on the attributes of a rack. The first decision tree depends on the attributes A1, A2, and A3 described above. The first decision tree outputs for each combination of the possible values of those attributes, a respective rack priority. In fig. 5a, the possible values of the rack priority are denoted by N1, N2, N3, N4, and N5, wherein N1 > N2 > N3 > N4 > N5. For instance, N1=11, N2=10, N3=9, N4=5, and N5=4.

Racks without any pending requests and stored in the storage unit 34 are assigned the lowest priority (N5). For these lowest priority racks, an instruction may be determined to keep these racks in the storage unit 34 and potentially to move them inside the storage unit 34 to areas of low priority or accessibility. Racks without any pending requests and located in the rack handling unit 32 are assigned a relatively low priority (N4), that priority being higher than the lowest possible priority (N5). Instructions for handling racks with this priority may therefore be prioritized over the racks with the lowest priority. Racks with attribute A3 equal to "Load to storage" can generally be assigned the highest priority (N1), ensuring that these racks are immediately handled. According to the first decision tree, racks with attribute A3 equals to "Retrieve from storage", are generally considered less urgent than the ones with attribute A3 equals to "Load to storage" and have rack priority equals to N2. Racks with attribute A3 equals to "Manual removal" or "Automatic disposal" are given a moderate priority, N3.

For example, with reference to Fig. 6a, if the laboratory device 30 is a storage device, the rack priority of the rack 40a is obtained by following the branch 501-502-503-504 and the rack priority of the rack 40f is obtained by following the branch 506-507-508. In some examples, the rack priority of the racks 40a-40j determined by using the first decision tree and the attributes listed in table 1a reads as follows:

**Table 2a**

| Rack | Rack priority | Rack | Rack priority |
|---|---|---|---|
| 40a | N1 | 40f | N5 |
| 40b | N4 | 40g | N5 |
| 40c | N3 | 40h | N2 |
| 40d | N2 | 40i | N3 |
| 40e | N1 | 40j | N5 |

**Fig. 5b** schematically depicts the second decision tree. The second decision tree depends on the attributes A1, A2, and A3. The second decision tree differs from the first decision tree in the output leaves 509a-509j of the branches, i.e., in the value of the rack priority. In particular, for each branch, the rack priority may be computed by using the mathematical formula in the last leave 509a-509j of that branch. In the mathematical formulas in the last leave 509a-509j of the second decision tree, the integer K is the number of pending requests of the rack. According to the formulas in the leaves 509a-509d and 509f-509i, the rack priority of a rack increases as the number of pending requests of that rack increases. Hence, racks with identical attribute A3 but different attribute A1 will have different rack priority, the rack with the higher number of pending requests having the highest rack priority. For example, with reference to the racks 40a-40j contained in the storage device in Fig. 6a, the rack priority of the rack 40a is obtained by following the branch 501-502-503-509f and is equal to 0.2 x 2 + 11 = 11.4. The rack priority of the rack 40e is obtained by following the branch 506-510-511-509a and is equal to 0.2x3+11=11.6.

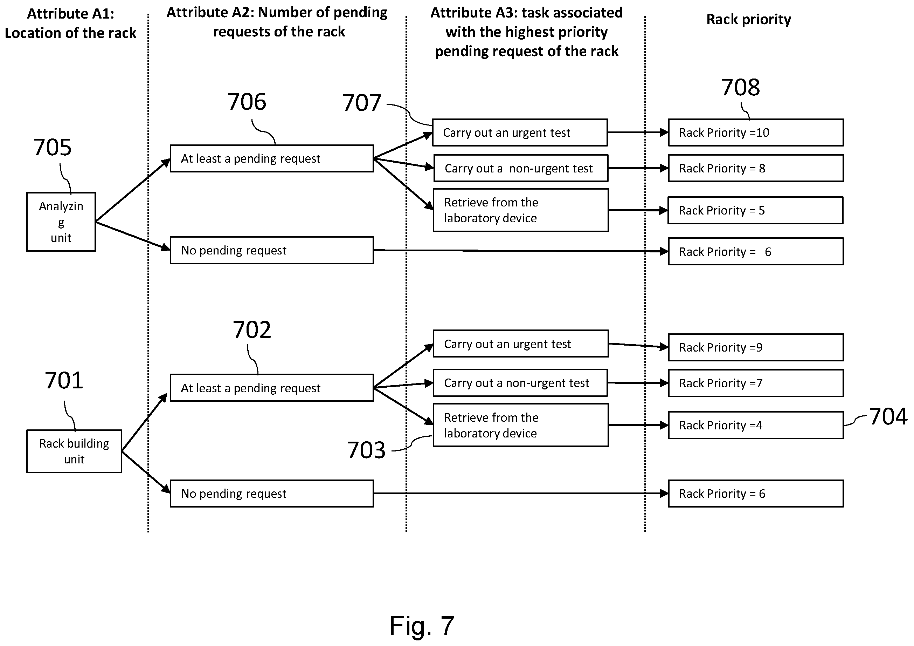

**Fig. 7** schematically depicts a third exemplary decision tree according to the present disclosure. In particular, this decision tree is configured to output the rack priority of a rack by using the attributes of that rack e.g., when the laboratory device 30 is an analyzer. The third decision tree depends on the attributes A1, A2, and A3 described above and outputs, for each combination of the possible values of those attributes, a respective rack priority. Racks without any pending requests are assigned a moderate priority (rack priority, RP = 6). Racks with attribute A3 equals to "Carry out an urgent test" are assigned the highest priorities, i.e., RP=10 or RP=9, depending on whether those racks are located in the analyzing unit 34 or in the rack building unit 32. Racks with attribute A3 equals to "Carry out a non-urgent test" are considered less urgent than the ones with attribute A3 equals to "Carry out an urgent test". Racks with attribute A3 equals to "Retrieve from the laboratory device" are given the lowest priorities, i.e., RP=5 or RP=4, depending on whether those racks are located in the analyzing unit 34 or in the rack building unit 32.

For example, with reference to Fig. 6a, if the laboratory device 30 is an analyzer, the rack priority of the rack 40a is obtained by following the branch 701-702-703-704 and the rack priority of the rack 40i is obtained by following the branch 705-706-707-708. In some examples, the rack priority of the racks 40a-40j determined by using the third decision tree and the attributes listed in table 1b reads as follows:

**Table 2b**

| Rack | Rack priority | Rack | Rack priority |
|---|---|---|---|
| 40a | 4 | 40f | 8 |
| 40b | 9 | 40g | 8 |
| 40c | 7 | 40h | 6 |
| 40d | 6 | 40i | 10 |
| 40e | 5 | 40j | 10 |

With reference to Fig. 4, the method 100 comprises determining 108, based on the rack priority data, a set of instructions for handling the set of racks contained in the laboratory device. These instructions may include transferring racks between the different units 32, 34 within the laboratory device 30 or between the automated laboratory system 10 and the laboratory device 30. Exemplarily, the set of instructions may comprise information specifying a first set of racks and a second set of racks, wherein the first set of racks consists of the racks of the first unit 32 that are to be moved to the second unit 34 and the second set of racks consists of the racks of the second unit 34 that are to be moved to the first unit 32. In particular, determining the set of instructions may comprise determining, among the set of racks, the N racks with highest rack priority, wherein N is a positive integer lower than or equal to the number of racks that the first unit may accommodate. determining the set of instructions may further comprise determining, among the N racks with highest rack priority, the second set of racks which consists of the Q racks comprised in the second unit, wherein Q is a positive integer lower than or equal to N. The first set of racks consists of the Q racks comprised in the first unit which are not among the N racks with highest rack priority.

In some examples, as discussed above, the laboratory device 30 schematically depicted in Fig. 6a may be a storage device containing the racks 40a-40d in the rack handling unit 32 and the racks 40e-40j in the storage unit 34. Moreover, the rack priorities of the racks 40a-40b may be the priorities listed in table 2a. If this is the case, determining the set of instructions may comprise determining, among the set of racks, the four racks with highest rack priority. Those four racks are determined by:
∘ ordering the racks 40a-40j according to the decreasing priority, i.e., 40a, 40e, 40d, 40h, 40c, 40i, 40b, 40f, 40h, 40g; and
∘ selecting the first four racks, i.e., 40a, 40e, 40d, 40h.

Exemplarily, if two or more racks have the same priority and are contained in different units, the rack in the rack handling unit 32 is considered to have the highest priority among the two or more racks. For instance, if the two or more racks have the same priority and are contained in the same unit, the ordering of those rack is obtained by randomly ordering those two or more racks.

In those examples, the set of racks to be moved from the storage unit 34 to the rack handling unit 32, i.e., the second set of racks, consists of the racks 40e and 40h. The racks to be moved from the rack handling unit 32 to the storage unit 34 are the two racks that are contained in the rack handling unit 32 and are not among the four racks with highest priority. Hence, those two racks are the racks 40c and 40b. Thus, the set of instructions specify that the racks 40c and 40b are to be moved from the rack handling unit 32 to the storage unit 34 and the racks 40e and 40h are to be moved from the rack handling unit 32 to the storage unit 34.

In some other examples, as discussed above, the laboratory device 30 schematically depicted in Fig. 6a is an analyzer containing the racks 40a-40d in the rack handling unit 32 and the racks 40e-40j in the analyzing unit 34. Moreover, the rack priorities of the racks 40a-40b may be the priorities listed in table 2b. In this case, determining the set of instructions may comprise determining, among the set of racks, the four racks with lowest rack priority by:
∘ ordering the racks 40a-40j according to the increasing priority, i.e., 40a, 40e, 40d, 40h, 40c, 40f, 40g, 40b, 40i, 40j; and
∘ selecting the first four racks, i.e., 40a, 40e, 40d, 40h.

If two or more racks have the same priority and are contained in different units, the rack in the rack handling unit 32 is considered to have the lowest priority among the two or more racks. For instance, if the two or more racks have the same priority and are contained in the same unit, the ordering of those rack is obtained by randomly ordering those two or more racks.

In those other examples, the set of racks to be moved from the analyzing unit 34 to the rack handling unit 32, i.e., the second set of racks, consists of the racks 40e and 40h. The racks to be moved from the rack handling unit 32 to the analyzing unit 34 are the two racks that are contained in the rack handling unit 32 and are not among the four racks with lowest priority. Hence, those two racks are the racks 40c and 40b. Thus, the set of instructions specify that the racks 40c and 40b are to be moved from the rack handling unit 32 to the analyzing unit 34 and the racks 40e and 40h are to be moved from the rack handling unit 32 to the analyzing unit 34.

Exemplarily, the method 100 may optionally comprise instructing 110 the laboratory device to handle the racks of the set of racks according to the set of instructions. In some cases, if the method is carried out by the computing device comprised in the storage device 30 schematically depicted in fig. 6a, the method 100 may optionally comprise instructing 110 the robotic arm 33 to handle the racks of the set of racks according to the set of instructions.

In particular, if the laboratory device is the storage device 30 schematically shown in Fig. 6a, the robotic arm 33 of the storage device 30 handles racks 40a-40j according to the set of instruction by moving the racks 40c and 40b from the rack handling unit 32 to the unit 34 and by moving the racks 40e and 40h from the rack handling unit 32 to the unit 34. After those racks are moved by the robotic arm 33, the racks 40a-40j contained in the storage device 30 are arranged as schematically shown in **Fig. 6b****.**

Fig. 6b provides a further schematic depiction of the storage device 30. In particular, Fig. 6a and fig. 6b differ from one another in the configuration of the racks 40a-40j. In the rack configuration depicted in fig. 6a, the four racks 40a, 40d, 40e, 40h are contained in the rack handling unit 32 and the six racks 40b, 40f, 40g, 40c, 40i, 40j are contained in the unit 34.

As used herein, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Further, as used herein, the phrase "at least one" or similar, e.g., "one or more of", in reference to a list of one or more entities should be understood to mean at least one entity selected from any one or more of the entities in the list of entities, but not necessarily including at least one of each and every entity specifically listed within the list of entities and not excluding any combinations of entities in the list of entities. This definition also allows that such entities may optionally be present other than the entities specifically identified within the list of entities to which the phrase "at least one" or similar refers, whether related or unrelated to those entities specifically identified. Thus, as a non-limiting example, "at least one of A and B" (or, equivalently, "at least one of A or B" or, equivalently "at least one of A and/or B" or, equivalently "one or more of A and B", "one or more of A or B", or "one or more of A and/or B") may refer, in one example, to at least one, optionally including more than one, A, with no B present (and optionally including entities other than B); in another example, to at least one, optionally including more than one, B, with no A present (and optionally including entities other than A); in yet another example, to at least one, optionally including more than one, A, and at least one, optionally including more than one, B (and optionally including other entities). In other words, the phrases "at least one," "one or more," and "and/or" are open-ended expressions that are both conjunctive and disjunctive in operation. For example, each of the expressions "at least one of A, B, and C," "at least one of A, B, or C," "one or more of A, B, and C," "one or more of A, B, or C," and "A, B, and/or C" may mean A alone, B alone, C alone, A and B together, A and C together, B and C together, A, B, and C together, and optionally any of the above in combination with at least one other entity.

As used herein, the phrase "being indicative of" may for example mean "reflecting" and/or "comprising". Accordingly, an entity, element and/or step referred to herein as "being indicative of [...]" can be synonymously or interchangeably used herein with one, two or all of said entity, element and/or step "comprising [...]" and said entity, element and/or step "reflecting [...]".

Further, as used herein, phrases such as "based on", "related" or "relating", "associated" and similar are not to be seen exclusively in terms of the entities, elements and/or steps to which they are referring, unless otherwise stated. Instead, these phrases are to be understood inclusively, unless otherwise stated, in that, for example, an entity, element or step referring by any of these phrases or similar, e.g., being "based on", an or another entity, element or step, does not exclude that the respective entity, element or step may be further or also "based on" any other entity, element or step than the one to which it refers.

Any designation of methods and steps as first, second, etc. as provided herein is merely intended to make the methods and their steps referenceable and distinguishable from one another. By no means does the designation of methods and steps constitute a limitation of the scope of this disclosure. For example, when this disclosure describes a third step of a method, a first or second step of the method do not need to be present yet alone be performed before the third step unless they are explicitly referred to as being required per se or before the third step. Moreover, the presentation of methods or steps in a certain order is merely intended to facilitate one example of this disclosure and by no means constitutes a limitation of the scope of this disclosure. Generally, unless no explicitly required order is being mentioned, the methods and steps may be carried out in any feasible order. Specifically, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

Any reference signs in the claims should not be construed as limiting the scope.

## Claims

1. A computer-implemented method comprising:
- obtaining a set of requests, wherein each request of the set of requests is a request to carry out an action on a respective sample tube and wherein each request of the set of requests is associated with a respective request priority for that request;
- determining, based on the set of requests, rack attribute data indicative, for each rack of a set of racks contained in a laboratory device, of a respective set of attributes of that rack, wherein each rack of the set of racks is configured to hold one or more sample tubes and holds or is assigned to hold a respective set of sample tubes, and;
- determining, based at least on the rack attribute data, rack priority data indicative, for each rack of the set of racks, of a respective rack priority of that rack; and
- determining, based on the rack priority data, a set of instructions for handling the set of racks contained in the laboratory device.

2. The method of claim 1, wherein the set of instructions comprises one or more of:
at least an instruction for transferring one or more racks of the set of racks between the automated laboratory system and the laboratory device;
at least an instruction for transferring the one or more racks of the set of racks within the laboratory device;
at least an instruction for transferring one or more racks of the set of racks between the laboratory device and another laboratory device;
at least an instruction to keep one or more racks of the set of racks in an idle state;
at least an instruction for removing or disposing sample tubes from one or more racks of the set of racks;
at least an instruction for moving one or more sample tubes from one rack of the set of racks to another rack of the set of racks;
at least an instruction for moving a sample tube from a single-tube holder to a rack of the set of racks; and
at least an instruction for moving a sample tube from a rack of the set of racks to a single-tube holder.

3. The method of claim 1 or 2, wherein the laboratory device comprises a first unit and a second unit, wherein the first unit of the laboratory device is configured to contain one or more racks of the set of racks so that sample tubes contained in at least one of those one or more racks are transferable between the automated laboratory system and the laboratory device, and wherein the second unit of the laboratory device is configured to contain one or more racks of the set of racks and to process and/or store sample tubes contained in those one or more racks.

4. The method of claim 3, wherein the set of instructions comprises one or more of:
at least an instruction for transferring one or more racks of the set of racks from the first unit to the second unit; and
at least an instruction for transferring one or more racks of the set of racks from the second unit to the first unit.

5. The method of any one of the previous claims, wherein, for each rack of the set of racks, the respective set of attributes comprises one or more of:
• an attribute indicative of whether at least one request of the set of requests is a request to carry out an action on a sample tube held or assigned to be held by said each rack;
• an attribute indicative of a cardinality of a respective subset of the set of requests, each request of the respective subset being a request to carry out an action on a respective sample tube held or assigned to be held by said each rack;
• an attribute indicative of a respective time at which a respective first request was obtained, wherein the respective first request is, among the requests of the respective subset, a request with highest priority; and
• an attribute indicative of the respective action associated with the respective first request.

6. The method of any one of the previous claims, wherein, determining the rack priority data comprises determining, for at least a rack of the set of racks, the respective rack priority such that:
- the respective rack priority when at least one request of the set of requests is a request to carry out an action on a sample tube held or assigned to be held by said rack is higher than the respective rack priority when no request of the set of requests is a request to carry out an action on a sample tube held or assigned to be held by said rack; and/or
- the rack priority of each rack such that at least one request of the set of requests is a request to carry out an action on a sample tube held or assigned to be held by said each rack is higher than the rack priority of each rack such that no requests of the set of requests is a request to carry out an action on a sample tube held or assigned to be held by said each rack; and/or
- the respective rack priority increases as the cardinality of the respective subset of the set of requests increases; and/or
- the respective rack priority increases as the respective time at which the respective first request was obtained decreases.

7. The method of any one of the previous claims, wherein determining the rack priority data comprises determining, for at least a rack of the set of racks, the respective rack priority such that:
- the respective rack priority when the respective action associated to the first request is the action of loading the respective sample tube to the laboratory device is higher than when the respective action associated to the first request is an action different from the action of loading the respective sample tube to the laboratory device; or
- the respective rack priority when the respective action associated to the first request is the action of retrieving the respective sample tube from the laboratory device is higher than when the respective action associated to the first request is an action different from the action of retrieving the respective sample tube from the laboratory device.

8. The method of any one of the previous claims, wherein the laboratory device is one or more of: a laboratory analyzer, a pre-analytic laboratory instrument, and a post-analytic laboratory instrument.

9. The method of any one of the previous claims, wherein the laboratory device is a storage device for storing sample tubes.

10. The method of any one of the previous claims, wherein
determining rack attribute data comprises determining, for each rack of the set of racks, the respective set of attributes of that rack, based on the request priority of each request of the set of requests which is a request to carry out an action on a sample tube of the respective set of tubes held or assigned to be held by that rack; and/or
determining rack priority data comprises determining, for each rack of the set of racks contained in the laboratory device, the respective rack priority of that rack based on the respective set of attributes of that rack.

11. The method of any one of the previous claims, further comprising causing the set of racks to be handled according to the set of instructions.

12. The method of claim 11, wherein causing the set of racks to be handled according to the set of instructions comprises one or more of: instructing the laboratory device and/or the automated laboratory system to handle the racks of the set of racks according to the set of instructions, and handling the racks of the set of racks according to the set of instructions.

13. A computer program comprising instructions which, when the program is executed by a computer, cause the computer to carry out the method according to any one of claims 1 to 12.

14. A computer-readable medium comprising instructions which, when executed by a computer, cause the computer to carry out the method according to any one of claims 1 to 12.

15. A data processing system comprising a processor configured to carry out the method according to any one of claims 1 to 12.
